(19) Europäisches Patentamt
European Patent Office
Office européen des brevets

(11) EP 0 557 950 B1

(12) # EUROPÄISCHE PATENTSCHRIFT

(45) Veröffentlichungstag und Bekanntmachung des Hinweises auf die Patenterteilung:
**30.10.1996 Patentblatt 1996/44**

(51) Int Cl.[6]: **C08G 65/40**

(21) Anmeldenummer: **93102846.8**

(22) Anmeldetag: **24.02.1993**

(54) **Polyetherketone und Polyethersulfone auf Basis von Phenylindan und ihre Verwendung für optische Systeme**

Polyetherketones and polyethersulphones based on phenylindane and their use for optical systems

Polyéthercétones et polyéthersulfones à base de phénylindane et leur utilisation pour des systèmes optiques

(84) Benannte Vertragsstaaten:
**BE DE FR GB IT NL SE**

(30) Priorität: **26.02.1992 DE 4205811**

(43) Veröffentlichungstag der Anmeldung:
**01.09.1993 Patentblatt 1993/35**

(73) Patentinhaber: **HOECHST AKTIENGESELLSCHAFT**
**65926 Frankfurt am Main (DE)**

(72) Erfinder:
- **Yang, Dazhong**
  **W-8580 Bayreuth (DE)**
- **Maier, Gerhard, Dr.**
  **W-8000 München 45 (DE)**
- **Nuyken, Oskar, Prof. Dr.**
  **W-8000 München 1 (DE)**
- **Brekner, Michael-Joachim, Dr.**
  **W-6000 Frankfurt am Main 1 (DE)**
- **Helmer-Metzmann, Freddy, Dr.**
  **W-6500 Mainz 1 (DE)**

(56) Entgegenhaltungen:
**WO-A-90/14378** **GB-A- 1 387 303**
**US-A- 3 065 205**

- **PATENT ABSTRACTS OF JAPAN, Band 10, Nr. 362 (C-389), 1986; & JP-61159421 (MITSUI TOATSU CHEM. INC.)**

EP 0 557 950 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft Polyaryletherketone und Polyethersulfone, die Phenylindan-Einheiten enthalten, ihre Herstellung und Verwendung für optische Systeme.

Transparente Thermoplasten finden zunehmend Anwendung in optischen Systemen der Leistungsoptik sowie in speziellen Optiken in Form von Linsen, Prismen, als Trägermaterial für verschiedene optische Schichten , als transparentes Beschichtungsmaterial von Spiegeln, Linsen und Prismen sowie als Lichtwellenleiter. Der allgemeine Vorteil von Thermoplasten ist ihre im Vergleich zu Glas relativ kostengünstige Verarbeitbarkeit und die für mobile Optiken besonders vorteilhafte, geringe Dichte. Bei optischen Teilen aus Thermoplasten entfallen oft die kostenintensiven Oberflächenbehandlungen, da diese Materialien in polierte Formen gegossen oder gespritzt werden können. Gläser mit einem Brechungsindex von ca. 1,5, z. B. Kronglas, liegen in der gleichen Preisklasse wie entsprechende, transparente Thermoplasten. Mit steigendem Brechungsindex werden die Gläser aber bedeutend teurer und damit steigen auch die Verarbeitungskosten bzw. die Kosten für die Oberflächenbearbeitung dieser Gläser, die immer mit Materialverlust verbunden ist. Folglich wird aus wirtschaftlicher Sicht eine Substitution von Glas durch Kunststoff mit steigendem Brechungsindex zunehmend attraktiv.

Die bisher für optische Anwendungen eingesetzten Standardkunststoffe wie Polymethylmethacrylat und Polycarbonat mit einem Brechungsindex von 1,49 bzw. 1,58 sind zwar leicht verarbeitbar, haben aber eine noch relativ niedrige Glastemperatur. Andere amorphe Hochleistungskunststoffe mit Brechungsindex von 1,6 bis 1,66 wie Polyarylethersulfone, Polyarylsulfone und Polyetherimide, welche alle sehr hohe Glastemperaturen besitzen (Tg ist größer als 180°C) und die entsprechend schwieriger zu verarbeiten sind, sind in den letzten Jahren auch für optische Anwendungen eingesetzt worden. (JP-OS 61-144 738, JP-OS 61-005 986, US-PS 4 477 555, EP-A 02 54 275, DE-OS 34 29 074). Auch auf dem Gebiet der Polyester sind Entwicklungen in Richtung amorpher Polyester mit hoher Glastemperatur zu verzeichnen, die ebenfalls für optische Anwendungen in Frage kommen. Der große Nachteil aller amorphen Polymere, welche Ester- oder Amidgruppen enthalten, ist deren geringe chemische Stabilität bzw. deren Hydrolyseempfindlichkeit.

Aus der internationalen Anmeldung WO 90/14378 sind bereits amorphe lineare aromatische Polyetherketone bekannt, die Bis-oxy-phenylindan- bzw. Bisoxy-tetramethyl-spirobiindan-Einheiten enthalten. Diese Polymeren waren transparent aber zeigten einen geringen Gelbstich.

Ausgehend von dem durch die erwähnten Thermoplasten abgedeckten Eigenschaftsspektrum wurde die Aufgabe gestellt, ein weiteres thermoplastisch verarbeitbares Polymer für optische Anwendungen bereitzustellen, das keine hydrolyseempfindliche Gruppen wie Ester- und Amidgruppen enthält. Die Wasseraufnahme sollte möglichst niedrig sein. Wegen des Einsatzes für optische Anwendungen sollte die Transparenz hoch und das Erscheinungsbild möglichst farblos sein. Die Glastemperatur sollte höher liegen als bei Polycarbonat. Gleichzeitig sollte das Polymer bei niedrigeren Temperaturen verarbeitet werden können als Hochleistungspolymere wie Polyetherimide und einen mit Polycarbonat und Polyetherimiden vergleichbaren Brechungsindex aufweisen.

Eigene durchgeführte Untersuchungen an unterschiedlichen Polymerklassen haben gezeigt, daß überraschenderweise bestimmte aromatische Polyether, welche neben den Ethergruppen in der Kette auch an Phenylindan-Einheiten gebundene Ketogruppen oder Sulfonylgruppen enthalten, dem geforderten Eigenschaftsprofil nahe kommen.

Handelsübliche Polyaryletherketone sind teilkristallin. Es handelt sich dabei um Polykondensate, deren Moleküle im wesentlichen unverzweigt und überwiegend aus unsubstituierten Phenyl- oder Biphenyleinheiten aufgebaut sind, die überwiegend para-ständig durch Ether- oder Carbonylgruppen miteinander verknüpft werden. Sie sind außerordentlich beständig gegen Lösemittel und chemischen Angriff und zeichnen sich in gefülltem Zustand durch hohe Wärmeformbeständigkeit aus.

Durch Störung der oben geschilderten relativ einfachen, sehr regelmäßigen Kettenstruktur kann die Kristallisationsneigung solcher Polyaryletherketone unterbunden werden. Beispielsweise erhält man durch Einbau eines erhöhten Anteils an ortho- bzw. meta-Verknüpfungen der Phenylringe oder durch Einbau von Einheiten, die substituierte aromatische Ringe enthalten, amorphe Polykondensate. Diese sind in herkömmlichen Lösemitteln (Chloroform, N,N-Dimethylacetamid, N,N-Dimethylformamid oder N-Methyl-2-pyrrolidon) löslich. Sie weisen im DSC-Diagramm nur eine Glastemperatur und keinen Schmelzpunkt auf. Aus Lösung und Schmelze bilden sie feste, flexible und transparente Filme. Anders als etwa die ebenfalls amorphen Polyarylethersulfone haben amorphe Polyetherketone bisher noch keine technische Bedeutung als Thermoplaste gewonnen.

Der Einsatz von organischen, hochmolekularen Materialien mit einer Hauptkettenstruktur aus aromatischen Ringen, beispielhaft sind u.a. Polyetherketone genannt, für optische Schaltkreiszubehörteile für nahe Infrarot-Strahlung ist bekannt (JP-OS Sho 61-208001). Zu diesen Teilen zählen Linsen, Prismen und Lichtwellenleiter. Nähere Angaben über die Zusammensetzung der hochmolekularen Materialien können aber der Veröffentlichung nicht entnommen werden.

Es bestand daher die Aufgabe, verbesserte Polymere bereitzustellen, die sich für optische Anwendungen eignen.

Die Erfindung betrifft daher polymere Ether mit Phenylindaneinheiten, die aus mindestens einer Struktureinheit

der Formel I

-O-A-O-Y- (I)

aufgebaut sind, wobei
A den Rest

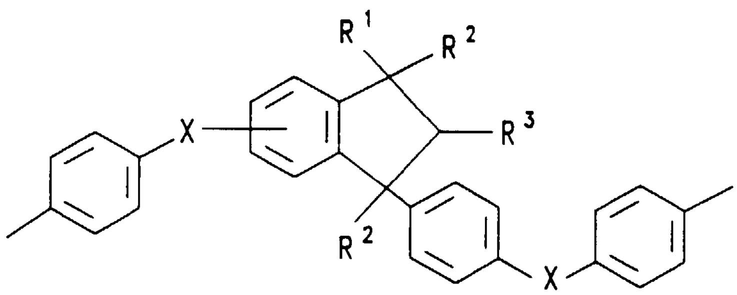

mit

X = CO oder $SO_2$,
$R^1 = CH_3$; $R^2 = R^3$ = H oder
$R^2 = CH_3$; $R^1 = R^3$ = ($C_1$ bis $C_{16}$)-Alkyl oder Aryl,
$R^1 = CH_3$; $R^3$ = H; $R^2$ = ($C_1$ bis $C_{16}$)-Alkyl oder Aryl

bedeutet und

Y einen divalenten Heteroarylen-Rest oder Arylenrest oder eine zweiwertige Gruppe aus zwei über eine Brücke miteinander verbundenen Arylresten bedeutet.

Beispiele für eine zweiwertige Gruppe, die aus zwei über eine Brücke miteinander verbundenen einwertigen Arylresten besteht, sind Reste, die sich vom Phenylindan ableiten, insbesondere die Reste

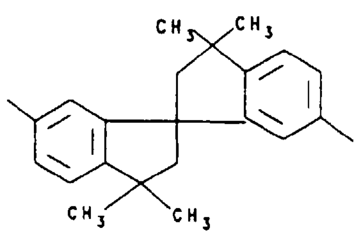

und

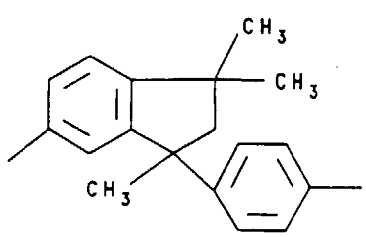

Hervorragende Polymere lassen sich auch herstellen, wenn Y gleich

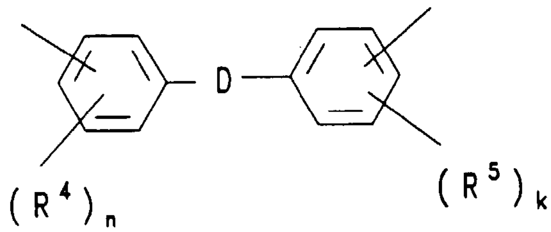

worin
$R^4$ und $R^5$ gleich oder verschieden sind und Halogen, vorzugsweise Cl oder Br, $C_1$-$C_4$-Alkyl- oder $C_1$-$C_4$Alkoxy-Gruppen oder Phenoxy-Gruppen darstellen, k und n gleich oder verschieden sind und Null oder eine ganze Zahl von 1-4 bedeuten, und D eine Ketogruppe, eine Terephthaloyl-Gruppe, einen Kohlenwasserstoff-Rest, der auch durch Trifluormethylgruppen substituiert sein kann, einen Heterocyclus oder eine aus Schwefel und/oder Sauerstoff bestehende Gruppe bedeutet. Die Reste $R^4$ und $R^5$ befinden sich vorzugsweise in 3- oder 3,5-Stellung zu D.

Falls D einen Kohlenwasserstoff-Rest bedeutet, der auch durch Trifluormethylgruppen substituiert sein kann, wird D vorzugsweise ausgewählt aus.

$D^1$ $-CH_2-$, $D^2$ $-C(CH_3)_2-$, $D^3$ $-C(CF_3)_2-$

$D^4$ 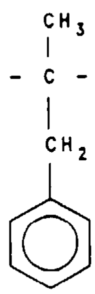 $D^5$ 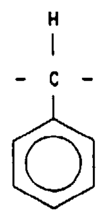 $D^6$ 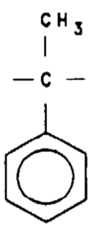

$D^7$ 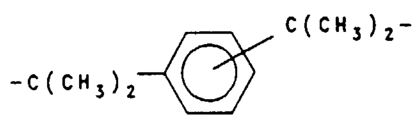 (m oder p)

$D^8$ 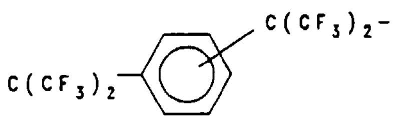 (m oder p)

$D^9$ 1,1-Cyclohexylen

$D^{10}$ 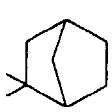 $D^{11}$ 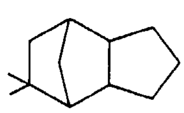 $D^{12}$ 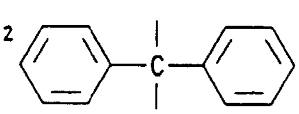

$D^{13}$ 9,9-Fluorenylen

Falls D einen Heterocyclus bedeutet, sind Furylen-, Oxadiazolylen- oder Thiadiazolylen-Reste bevorzugt.

Falls D aus Schwefel und/oder Sauerstoff besteht, sind im wesentlichen die Gruppen -S-, -O-, -SO- und $-SO_2-$ von Interesse.

Hervorragende Polymere werden auch erhalten, wenn der Rest Y einen Arylenrest darstellt.

Dabei kann Y z.B ein 1,5-, ein 1,8-, ein 2,6- oder ein 9,10-Anthracenylen-Rest, ein Fluorenylen-Rest, ein 1,5-, ein 2,6-, ein 2,7-Naphthylen-Rest sein. Die beiden freien Valenzen können auch am gleichen Ring des Naphthalin-Skeletts vorhanden sein. Bevorzugt sind die Reste

$Y^1$ 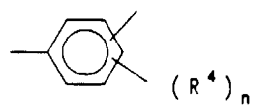

$Y^2$ 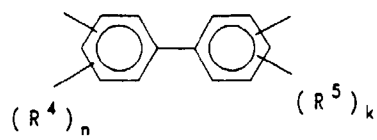

worin

$R^4$ und $R^5$ gleich oder verschieden sind und Halogen vorzugsweise Cl und Br, $C_1$-$C_4$-Alkyl- oder $C_1$-$C_4$Alkoxy-Gruppen oder Phenoxy-Gruppen darstellen, k und n gleich oder verschieden sind und Null oder eine ganze Zahl von 1 bis 4 bedeuten. Im Rest $Y^1$ kann $R^4$ auch für Phenyl stehen. Vorzugsweise sind k und n Null, 1 oder 2, insbesondere Null oder 2. Falls $R^4$ und $R^5$ Halogen bedeuten, sind k und n vorzugsweise 2. Besonders bevorzugt sind der o-, m- und p-Phenylenrest.

Falls Y einen divalenten Heteroarylen-Rest bedeutet, so sind die Reste 2,5-Pyridylen, 2,5-Furylen, 2,5-Oxadiazolylen, 2,5-Oxazolylen und 2,5-Thiadiazolylen bevorzugt.

Die Alkylreste $R^1$, $R^2$ und $R^3$ an den Phenylindaneinheiten A können linear oder verzweigt, monocyclisch oder polycyclisch sein. Sie enthalten vorzugsweise 1 bis 4 C-Atome, insbesondere ein C-Atom. Besonders bevorzugt ist es, wenn $R^1 = R^2 = CH_3$ und $R^3 = H$.

Die Arylreste $R^1$, $R^2$ und $R^3$ an den Phenylindaneinheiten A sind vorzugsweise Phenylreste.

Die erfindungsgemäßen Polymere lassen sich dadurch herstellen, daß man eine Halogenverbindung der allgemeinen Formel II

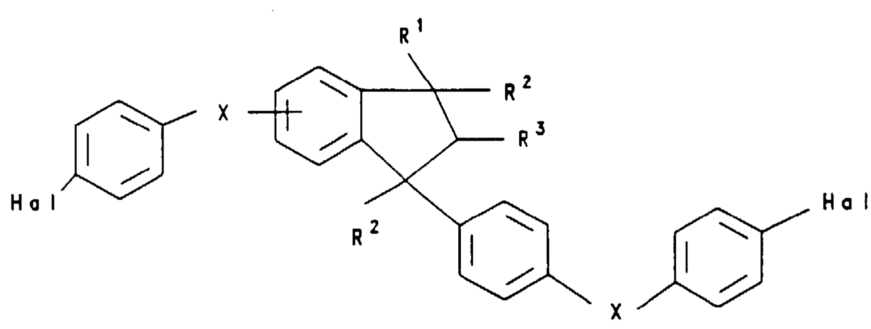

wobei

| | |
|---|---|
| Hal | für Chlor, Fluor, Brom oder Jod |
| X | für CO oder $SO_2$ steht und |
| $R^1$, $R^2$ und $R^3$ | die oben angegebene Bedeutung aufweisen, |

mit der etwa äquimolaren Menge einer aromatischen oder heteroaromatischen Dihydroxyverbindung HO-Y-OH, wobei Y die oben angegebene Bedeutung aufweist, in einem aprotisch-dipolaren Lösungsmittel umsetzt.

Bei dieser nukleophilen Polykondensation sind die aus dem Bisphenol durch Einwirken von Alkalien gebildete Phenolationen das eigentliche Agens (DE-PS 1545 106 und CA-PS 847 953), das die durch paraständige Gruppen X aktivierten Halogenatome der Verbindung II substituiert.

Dabei beträgt das molare Verhältnis der Monomeren (II) und $Y(OH)_2$, die zu den Einheiten A und Y führen, im allgemeinen 0,95 bis 1,05 zu 1,0 vorzugsweise 1:1. Bei Polymeren, die unter Einsatz von Dihydroxy-Indanverbindungen hergestellt worden sind, beträgt dieses molare Verhältnis im allgemeinen 1,001 bis 1,06 zu 1, vorzugsweise 1,002 bis 1,05 zu 1 und insbesondere 1,004 bis 1,05 zu 1.

Die aufgeführten Polymeren können Homopolykondensate sein, die also nur je eine Einheit vom Typ A und eine Einheit vom Typ Y je wiederkehrender Einheit enthalten, oder Copolykondensate, welche zwei oder mehrere verschiedene Einheiten vom Typ A und/oder zwei oder mehrere verschiedene Einheiten vom Typ Y enthalten.

Zusätzlich zu einem Homo- oder einem Copolykondensat können auch Polymermischungen eingesetzt werden, die aus zwei oder mehr der oben erwähnten Homopolykondensate, aus zwei oder mehr der erwähnten Copolykondensate oder aus mindestens einem Homo- und mindestens einem Copolykondensat bestehen.

Aus den erfindungsgemäß eingesetzten Polyaryletherketonen und -sulfonen (I) können Elemente für optische Systeme hergestellt werden. Unter dem Ausdruck "Elemente optischer Systeme" werden optische Geräteteile, Teile aus Spezialoptiken wie auch aus Systemen aus dem Bereich der Leistungsoptik, Lichtleitung und Optoelektronik verstanden, z.B. Linsen, Prismen, Beleuchtungs- und Projektionssysteme, Lichtleitsysteme, optische Beschichtungen und optische Träger.

Als Voraussetzung dafür, daß ein Thermoplast transparent ist, gilt die Abwesenheit intrinsischer Absorption in dem betreffenden Wellenlängenbereich und die Abwesenheit von Brechungsindexfluktuationen, die merkliche Streuverluste hervorrufen. Brechungsindefluktuationen werden entweder durch physikalische Zweiphasigkeit, d.h. Teilkristallinität, oder durch chemische Zweiphasigkeit, d.h. Entmischung, hervorgerufen. Verunreinigungen können sich sowohl als Absorptions- wie auch als Streuzentren negativ auf die Transparenz von Materialien auswirken, sind aber im Gegensatz zu den vorher erwähnten Faktoren mittels technologischer Maßnahmen, die sich sowohl auf die Polymersynthese als auch auf die Polymerverarbeitung beziehen, zu beseitigen.

Die erfindungsgemäßen polymeren Ether (mit X = CO) lassen sich prinzipiell auch durch eine elektrophile Polykondensation nach Friedel-Crafts aufbauen, wobei man die entsprechenden Phenylindan-dicarbonsäurehalogenide mit den entsprechenden Phenylethern der Bishydroxyverbindungen $Y(OH)_2$ umsetzt. In Anlehnung an den Stand der Technik können dabei unterschiedliche Reaktionsbedingungen gewählt werden (vgl US-PS 3 065 205, US-PS 3 441 538, Brit. Pat. Appl. 1 387 303, WO 84/3 8991).

Als aromatische Dihydroxyverbindungen $Y(OH)_2$ kommen z. B. einkernige Diphenole der Formel

III

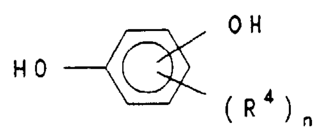

wie Hydrochinon, Resorcin oder deren Homologe, wie Methylhydrochinon, 2,3- und 2,5-Dimethylhydrochinon, 2,3,5,6-Tetramethylhydrochinon sowie 2-Methylresorcin in Betracht. Bevorzugt werden Hydrochinon, Resorcin, 2,3- und 2,5-Dimethylhydrochinon. geeignete mehrkernige Dihydroxyverbindungen sind beispielsweise solche, in denen zwei alkyl- oder alkoxy-substituierte oder unsubstituierte Phenolreste durch eine direkte Bindung oder durch Atome bzw. Gruppen wie Alkyliden, Sauerstoff oder Carbonyl verknüpft sind. Die geeigneten Verbindungen lassen sich teilweise durch die Formel IV

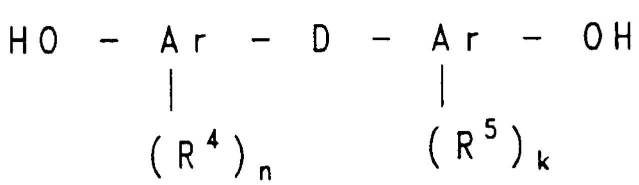

(IV)

beschreiben, wobei Ar für einen Arylrest, vorzugsweise Phenylen, steht, der durch Gruppen $R^4$ und $R^5$ substituiert sein kann. $R^4$ und $R^5$ stehen für Halogen, vorzugsweise Brom oder Chlor, Alkylgruppen oder Alkoxygruppen mit jeweils 1 bis 8, vorzugsweise 1 bis 4 C-Atomen und Aryl- oder Aryloxygruppen (Aryl vorzugsweise Phenyl). k und n sind gleich oder verschieden und im allgemeinen ganze Zahlen von Null bis 4, vorzugsweise Null, 1 oder 2, insbesondere Null oder 2. D ist z.B. eine Alkylengruppe, insbesondere mit 1 bis 3 C-Atomen, die gegebenenfalls mit Halogen, vorzugsweise Fluor, oder mit einem Arylkern substituiert ist, oder eine $C_6$-$C_{10}$-Cycloalkylidengruppe oder eine direkte Bindung und schließt die Gruppierungen ein: -O-, -C=O,

$-CH_2-$ , $-C(CH_3)_2-$, $-C(CF_3)_2)-$, 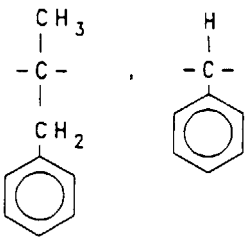 ,

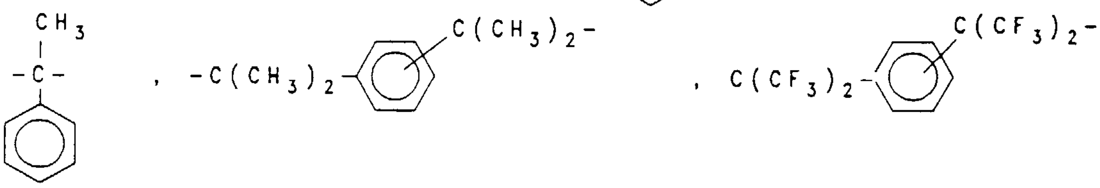

Entsprechende Verbindungen IV lassen sich aus gegebenenfalls substituierten Phenolen und Oxoverbindungen wie Aceton, Formaldehyd, Cyclohexanon, Heptanon-2, Octanon-2 etc. herstellen.

Beispiele für derartige mehrkernige Dihydroxy-Verbindungen sind Bis(4-hydroxyphenyl)methan, 3,4'-Dihydroxydiphenyl, 4,4'-Dihydroxybenzophenon, 4,4'-Dihydroxydiphenylether, Bis-(3,5-dimethyl-4-hydroxyphenyl)methan, 2,2-Bis(4-hydroxyphenyl)phenylpropan, 1,1-Bis(4-hydroxyphenyl)phenylethan, 1,3-Bis(4-hydroxyphenylisopropyliden)benzol, 1,3-Bis(3,5-dimethyl-4-hydroxyphenylisopropyliden)benzol, 1,3-Bis(4-hydroxyphenyl-1,1,1,3,3,3-hexafluorisopropyliden)benzol, 1,3-Bis(3,5-dimethyl-4-hydroxyphenyl-1,1,1,3,3,3-hexafluorisopropyliden)benzol, 2,7-Dihydroxynaphthalin, 2,3-Dihydroxynaphthalin, 1,4-Dihydroxynaphthalin, 2,7-Dihydroxy-9-fluorenon, sowie die bevorzugt eingesetzten Verbindungen 2,2-Bis(4-hydroxyphenyl)propan, (Bisphenol A), 2,2-Bis(3,5-dibrom-4-hydroxyphenyl)propan, 2,2-Bis(4-hydroxyphenyl)-1,1,1,3,3,3-hexafluorpropan, 3,3'-Dihydroxydiphenyl, 4,4'-Dihydroxydiphenyl, 3,3',5,5'-Tetramethyl-4,4'-Dihydroxydiphenyl, 3,3',5,5'-Tetramethyl-4,4'-Dihydroxybenzophenon, 2,2-Bis(3,5-dimethyl-4-hydroxyphenyl)-1,1,1,3,3,3-hexafluorpropan, 1,4-Bis(4-hydroxyphenylisopropyliden)benzol, 1,4-Bis(3,5-dimethyl-4-hydroxyphenylisopropyliden)benzol, 1,4-Bis(4-hydroxyphenyl-1,1,1,3,3,3-hexafluorisopropyliden)benzol, 1,4-Bis (3,5-dimethyl-4-hydroxyphenyl-1,1,1,3,3,3-hexafluorisopropyliden)benzol, 1,5-Dihydroxynaphthalin, 2,6-Dihydroxynaphthalin, 5-Hydroxy-3-(4-hydroxyphenyl)-1,1,3-trimethylindan, 5-Hydroxy-3-(3,5-dimethyl-4-hydroxyphenyl)-1,1,3,4,6-pentamethylindan, 6,6'-Dihydroxy-3,3,3',3'-tetramethyl-1,1'-spirobiindan. Die Herstellung von indanhaltigen Bisphenolen $Y(OH)_2$ wird beschrieben in der internationalen Anmeldung WO90/14378.

Die aktivierten aromatischen Dihalogenverbindungen lassen sich aus dem Phenylindan der Formel

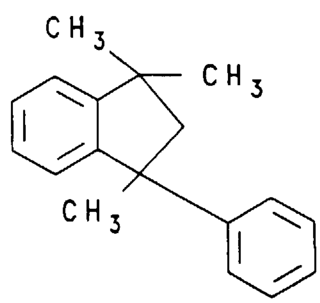

durch Reaktion mit einem Säurehalogenid der Formel Hal-p.$C_6H_4$-X-$Hal^1$ in einem Lösungsmittel in Gegenwart von $AlCl_3$ gewinnen. Dabei bedeuten Hal und $Hal^1$ unabhängig voneinander Chlor oder Fluor und X bedeutet $SO_2$ oder CO. Es entsteht ein Isomerengemisch, wobei die 5- und 6-substituierten Phenylindane anfallen. Durch Umkristallisieren

kann das 6-Isomere rein gewonnen werden. Das reine 6-Isomere läßt sich auch gewinnen aus dem 3-Phenylindan-6,4'-bissäurehalogenid mit überschüssigem Halogenbenzol in Gegenwart von $AlCl_3$.

Die Kondensationsreaktion wird entweder in Substanz oder in Gegenwart eines inerten Lösungsmittels durchgeführt, in denen das gebildete Polymere bei der Reaktionstemperatur löslich ist. Als Lösungsmittel kommen beispielsweise in Frage: Diphenylsulfon, N-Cyclohexyl-2-pyrrolidon, cyclische aromatische Sulfone wie Dibenzothiophen-S,S-dioxid oder, weniger bevorzugt, Benzophenon und cyclische aromatische Ketone, wie 9-Fluorenon. Derartige Lösungsmittel sind u.a. in der DE-OS 2 803 873 beschrieben.

Ebenfalls kommen als Lösungsmittel in Frage N,N-Dimethylformamid, N,N-Dimethylacetamid oder N-Methyl-2-pyrrolidon. In diesem Falle ist dem Reaktionsgemisch ein geeignetes Wasserschleppmittel (z.B. Toluol) hinzuzufügen.

Die Reaktionstemperatur ist nach unten durch die Schmelztemperatur mindestens einer der Komponenten oder des Lösungsmittels und nach oben durch die Zersetzungstemperatur der Kondensationspartner bzw. des gegebenenfalls verwendeten Lösungsmittels begrenzt. Sie liegt im allgemeinen zwischen 100 und 400 °C, vorzugsweise zwischen 150 und 350 °C, und hängt u.a. von der Reaktivität der Kondensationspartner und der Art der verwendeten Lösungsmittel ab. Vorzugsweise beginnt man mit einer niedrigen Reaktionstemperatur und steigert die Temperatur allmählich oder stufenweise, wenn die Viskosität der Reaktionsmasse steigt.

Die inhärente Viskosität bestimmt nach DIN 51562 der erfindungsgemäßen Polymere, als Maß für ihr Molekulargewicht, gemessen in einer Lösung von 0,1 g des Polymeren in 100 ml Chloroform bei 25 °C, kann in dem weiten Bereich von 0,2-2,5 dl/g, bevorzugt 0,3-1,5 dl/g, liegen.

Die Transparenz der erfindungsgemäßen Polymeren ist hoch und hängt in begrenztem Maße von der Anwesenheit von Verschmutzungen ab, die in Anhängigkeit von der Herstellungsmethode als Restsalz oder Restlösungsmittel vorliegen können. Entsprechende Reinigungschritte, die beispielsweise in wiederholtem Auswaschen, wiederholtem Umfällen, aber auch im Filtrieren der Polymerlösung bzw. der Polymerschmelze bestehen können, verbessern die Transparenz der amorphen Polyaryletherketone. Die Transparenz bzw. die Sauberkeit des Materials ist immer in Verbindung mit konkreten Anwendungen zu beurteilen. Folglich impliziert z.B. die Anwendung als Lichtwellenleiter viel aufwendigere Reinigungsprozeduren als die Anwendung als Spiegelsubstrat.

Die erwähnten Polymeren sind amorph. Ihre Glastemperaturen liegen gemäß DSC-Messungen (Differntial Scanning Calorimetry) bei 20°C/Min. oberhalb 155 °C und damit oberhalb der Glastemperatur von Polycarbonat. Bevorzugt werden Polymere mit Glastemperaturen von oberhalb 180°C.

Die Verarbeitungstemperaturen für Extrusion liegen zwischen 230 und 350 °C, bevorzugt zwischen 230 und 330°C und besonders bevorzugt zwischen 235 und 320 °C.

Die Dichten der amorphen Polyetherketone liegen generell unterhalb derer von analogen Polyethersulfonen. Je nach gewähltem Kettenaufbau werden Dichten zwischen 1,2 und 1,4 $g/cm^3$ gemessen. Bevorzugt werden Werte für die Dichte von unterhalb 1,35 $g/cm^3$.

Übliche Hilfsmittel wie Stabilisatoren, UV-Absorber, Aufheller, Entformungsmittel und Antistatika können ohne Beeinträchtigung der beschriebenen Eigenschaften eingearbeitet werden.

Die hohe Glastemperatur und die relativ leichte Verarbeitbarkeit führen zu einem effizienten Einsatz dieser Polymere im Bereich der Leistungsoptik, wo heute besonders bei Beleuchtungsoptiken und Projektionsoptiken immer höhere Energiedichten angestrebt werden, die dann auch eine höhere thermische Belastbarkeit der durchstrahlten optischen Elemente fordern. Beispielsweise sind Linsen oder Streuscheiben aus den erfindungsgemäßen Polymeren bei solchen Anwendungen denen aus Polymethylmethacrylat, Polystyrol oder Polycarbonat überlegen.

Die Spritzgießbarkeit der erfindungsgemäßen Polymere bietet besondere Vorteile bei der Herstellung von optischen Elementen für spezielle Lichtdesigns, womit gleichzeitig sowohl eine einfach oder mehrfach gebündelte wie auch eine gestreute Lichtverteilung angestrebt wird. Solche optische Elemente haben sehr komplexe geometrische Formen, die in der notwendigen Oberflächenqualität und mit vertretbaren Kosten fast nur noch aus Kunststoffen hergestellt werden können. Die lokale Erwärmung in diesen optischen Teilen, der die erfindungsgemäßen Polymeren ohne weiteres standhalten, überschreitet oft die thermische Belastbarkeit der transparenten Standardkunststoffe.

Auch im Bereich der Lichtleitung über kurze Strecken, die eine besonders thermische Belastung erfahren, bieten die erfindungsgemäßen Thermoplasten einige Vorteile. Außer der Verarbeitbarkeit mittels Extrusion zu Folien oder Fasern führt der relativ hohe Brechungsindex zu einer sehr guten Lichtbündelung und somit zu einer Eignung als Kernmaterial für lichtleitende Fasern bzw. Folien. Die Folien und Fasern aus diesen amorphen Polyetherketonen können auch in verhältnismäßig großen Dicken bzw. Durchmessern als Lichtleiter eingesetzt werden. Polyaryletherketone mit Brechungsindices unter 1,65 können auch als Mantelmatertial für Lichtwellenleiter eingesetzt werden. Bevorzugt werden Polymere mit Brechungsindices unter 1,63. Für Lichtwellenleiter mit Polymer-Kernen, die einen sehr hohen Brechungsindex aufweisen, bieten sich sehr viele Thermoplasten als Mantelmaterial an, z.B. Polyarylate, Polysulfone, Polyacrylate, Polymethacrylate, Vinylpolymere. Als Mantelmaterial kommen auch Kunststoffe mit einer noch höheren thermischen Formbeständigkeit als die erfindungsgemäßen Polymeren in Frage, z.B. Polyethersulfone, wodurch dann thermische Formbeständigkeit des betreffenden Lichtleiters mit einem Kern aus Polyaryletherketon noch weiter erhöht

wird. Auch lassen sich Lichtleiter herstellen, wo Mantel und Kern aus indanhaltigen Polymeren bestehen, die sich im Brechungsindex um Werte von mehr als 0,01 unterscheiden. Wegen der dabei erzielten guten Haftung zwischen Kern und Mantel sind solche Lichtleiter besonders bevorzugt.

Der hohe Brechungsindex der erfindungsgemäß eingesetzten amorphen Polymeren bietet auch ganz allgemein den Vorteil, daraus dünne Linsen mit hoher Brechkraft herzustellen. Auch ist es sehr vorteilhaft, die erfindungsgemäßen Thermoplasten bei der Herstellung von Achromaten zu verwenden. Dabei kommt nicht nur der hohe bzw. variable Brechungsindex zur Geltung, sondern auch die variable Abbe-Zahl. Beispielsweise wird bei Negativlinsen ein Polyaryletherketon mit niedriger Abbe-Zahl und bei Positivlinsen ein Polyaryletherketon mit hoher Abbe-Zahl bevorzugt. Die Abbe-Zahl kennzeichnet die Dispersion eines optischen Mediums. Diese ist definiert als

$$\nu = \frac{n_D-1}{n_F-n_C}$$

wobei $n_D$, $n_F$, $n_C$ die Brechungsindices des Mediums bei der Helium-D- und den Fraunhoferschen Linien F bzw. C bedeuten. Ihr Wert hängt von den Bauelementen der Polymerkette ab und liegt im Bereich zwischen 18 und 40, vorzugsweise 19 und 32 und besonders bevorzugt zwischen 20 und 30. Die bei der Herstellung von Achromaten verwendeten Asphären aus transparenten indanhaltigen Polyetherketonen und Polyethersulfonen lassen sich einzeln oder durch direktes Umspritzen der zu korrigierenden Glaslinse mit Polymer herstellen.

Durch die Transparenz, die relativ leichte Verarbeitbarkeit, die erhöhte Glastemperatur als auch durch andere Eigenschaften wie die geringe Wasseraufnahme, Hydrolysebeständigkeit und Unlöslichkeit in unpolaren und wenig polaren Lösemitteln, wie in aliphatischen Kohlenwasserstoffen, Alkoholen, in Diethylether, in Aceton u.a., eignen sich die erfindungsgemäßen Polymeren für die Verwendung als Träger von optischen Schichten. Solche Träger zeichnen sich dadurch aus, daß sie von dem auf die optische Schicht fallenden Licht durchstrahlt werden, daß sie bei der Beschichtung günstige Oberflächeneigenschaften, wie Unlöslichkeit, thermische Formbeständigkeit und chemische Resistenz aufweisen und daß die Transparenz des Trägers durch den Beschichtungsprozeß nicht verringert wird. Als optische Schicht kommen sowohl organische als auch anorganische Schichten in Frage, z.B. Farbstoffschichten, dielektrische Schichten oder metallische Schichten. Bevorzugt werden Träger optischer Schichten aus amorphen indanhaltigen Polymeren in Form von Platten und Folien, die beispielsweise als Substrate für optische Datenspeicher Einsatz finden.

Die Hydrolysebeständigkeit der amorphen indanhaltigen Polymeren wird genützt bei Optiken, die in korrosiven Medien verwendet werden. Es können sowohl ganze optische Elemente aus den erfindungsgemäßen Thermoplasten hergestellt werden, als auch gewisse optische Elemente aus Glas, Metall oder Kunststoff, wie beispielsweise Spiegel oder Linsen, die einem korrosiven Medium ausgesetzt sind, mit indanhaltigen Polyethersulfonen oder Polyetherketonen beschichtet werden. Die Beschichtung erfüllt dann hauptsächlich eine Schutzfunktion (Glas wird von einigen hauptsächlich sauren Medien auch angegriffen), kann aber auch optische Funktionen übernehmen, wie z.B. die Beeinflussung der Reflektivität von Oberflächen.

Zur Beschichtung können die erfindungsgemäßen Polymeren sowohl in Form von Folien aufgeklebt oder gepreßt werden als auch aus Lösung verarbeitet werden. Lösungsmittel, die für die Herstellung von Beschichtungslösungen aus erfindungsgemäßen amorphen Polymeren verwendet werden können sind: N-Methyl-2-pyrrolidon, Dimethylsulfoxid, N,N-Dimethylacetamid, N;N-Dimethylformamid. Die Beschichtung kann durch Aufschleudern oder über einen Tauchvorgang erfolgen. Anschließend wird die Beschichtung bei 100 bis 140 °C im Luftstrom getrocknet.

Beispiel 1:

Poly-(oxyphenylen-bismethylmethylen-phenylenoxyphenylen-carbonylphenylen-1,1,3-trimethyl-indanylen-5-carbonylphenylen)

In einem 2-Liter-Vierhalskolben mit Rührer, Rückflußkühler, Wasserabscheider und Innenthermometer wurde 0,44 mol BFBPI (=4,6-Bis-(4-fluorbenzoyl)-1,3,3-trimethyl-1-phenyl-indan) und 0,44 mol Bisphenol A in 1,5 l N,N-Dimethylacetamid und 300 ml Toluol gelöst, wobei der Kolben dauernd mit Inertgas gespült wurde. Nach dem vollständigen Auflösen wurden 62,18 g wasserfreie Soda zugegeben und die Mischung solange unter Rückfluß gerührt, bis im Wasserabscheider kein weiteres Wasser abgeschieden wurde (ca.12 h). Die Lösung wurde noch weitere 3 Stunden unter Rückfluß behandelt, abgekühlt und in 10%ige Essigsäurelösung eingegossen. Die polymere Titelverbindung wurde mehrfach mit Wasser/ Methanol und anschließend mit Aceton gewaschen und anschließend 12 h bei 140°C getrocknet. Das erhaltene Pulver (100,4 g) hatte eine inhärente Viskosität von 1,02 dl/g (Chloroform; 0,1 g/dl; 25°C) und eine $T_g$ von 204,5°C. Nach Schmelzen und Erstarren ist das Material transparent.

Beispiel 2:

Herstellung von Zwischenprodukten der Formel $AF_2$

20 g (84.6 mmol) 1,1,3-Trimethyl-3-phenylindan werden mit 27 g /170 mmol) 4-Fluorbenzoesäurechlorid in Gegenwart von 29.3 g (220 mmol) $AlCl_3$ in 100 ml Nitrobenzol als Lösungsmittel für 12 h zum Sieden erhitzt. Anschließend wird das Lösungsmittel im Vakuum entfernt und zum Rückstand langsam 500 ml 10%ige HCl gegeben. Diese Mischung wird mehrmals mit insgesamt 500 ml Chloroform extrahiert, um das Rohprodukt aufzunehmen. Die organische Phase wird mit Wasser gewaschen, im Vakuum eingeengt, und durch zweifache Säulenchomatographie (Kieselgel, Laufmittel Hexan/Ethylacetat 4:1) vorgereinigt. Durch Umkristallisieren aus Ethanol/Wasser (10:1) wird das Monomer in genügender Reinheit für die Polymerisation erhalten.
Ausbeute: 50%, Fp: 45-48°C.
Dieses Produkt erhält folgende beiden Isomere im Verhältnis 1:1:

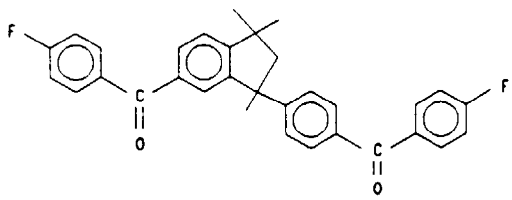

5-Isomer

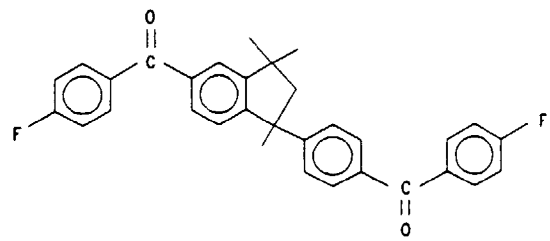

6-Isomer

Durch Umkristallisieren kann das 5-Isomer rein erhalten werden.

Beispiel 3:

Herstellung eines Zwischenproduktes

Die Darstellung erfolgt völlig analog zu Beispiel 2 aus 1,1,3-Trimethyl-3-phenylindan und 4-Chorbenzolsulfonylchlorid, jedoch fällt hier bereits nach einmaligem Umkristallisieren das 5-Isomer rein an.
Ausbeute: 35%, Fp: 200-202°C

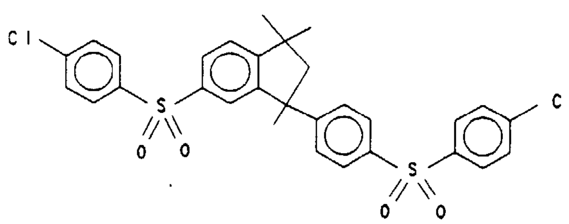

Beispiel 4:

Herstellung eines Zwischenproduktes

Die Darstellung erfolgt völlig analog zu Beispiel 2 aus 1,1,3-Trimethyl-3-phenylindan und 4-Fluorbenzolsulfonylchlorid, jedoch fällt hier bereits nach einmaligem Umkristallisieren das 5-Isomer rein an.
Ausbeute: 35%, Fp: 168-170°C

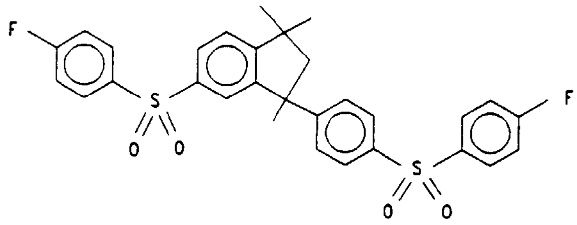

Beispiele für die Polymerisation:

Beispiel 5:

Poly-(oxyphenylen-oxyphenylen-oxyphenylen-carbonylphenylen-1,2,3-trimethylindanylen-carbonylphenylen)

In einem Kolben mit Wasserabscheider werden 600 mg (1,2 mmol) Monomer von Beispiel 2 (Isomerenmischung), 243 mg (1,2 mmol) 4,4'-Dihydroxydiphenylether und 249 mg (1,8 mmol) $K_2CO_3$ in einer Mischung aus 100 ml NMP und 50 ml Toluol für 20 h auf 190 °C erhitzt. Das Polymer wird durch Eingießen der Lösung in 500 ml Methanol gefällt, in Tetrahydrofuran gelöst und erneut mit Methanol gefällt, abfiltriert und im Vakuum getrocknet.
Ausbeute: 80%
Molmasse $M_n$ = 12000; $M_w$=21000
$T_g$ = 193°C $T_d$ = 438°C (Beginn der Gewichtsabnahme in Luft, Heizrate 10 K/min).

Beispiel 6:

In einem Kolben mit Wasserabscheider werden 1,0 g (2.08 mmol) Monomer von Beispiel 2 (Isomerenmischung), 387 mg (2,08 mmol) 4,4'-Dihydroxydiphenyl und 431 mg (3.12 mmol) $K_2CO_3$ in einer Mischung aus 140 ml N-Methylpyrrolidon (= NMP) und 70 ml Toluol auf 190 °C erhitzt. Nach 20 h wird das Polymer durch Eingießen der Lösung in 500 ml Methanol gefällt, in THF gelöst und erneut mit Methanol gefällt, abfiltriert und im Vakuum getrocknet.
Ausbeute: 90%
Molmasse $M_n$ = 13500; $M_w$=28400
$T_g$ = 219°C $T_d$ = 458°C (Beginn der Gewichtsabnahme in Luft, Heizrate 10 K/min).

Beispiel 7:

In einem Kolben mit Wasserabscheider werden 1,0 g (1.71 mmol) Monomer von Beispiel 3 (5-Isomer), 346 mg (1.71 mmol) 4,4'-Dihydroxydiphenylether und 355 mg (2.57 mmol) $K_2CO_3$ in einer Mischung aus 140 ml NMP und 70 ml Toluol für 20 h auf 190 °C erhitzt. Das Polymer wird durch Eingießen der Lösung in 500 ml Methanol gefällt, in THF gelöst, erneut mit Methanol gefällt, abfiltriert und im Vakuum getrocknet.
Ausbeute: 70%
Molmasse $M_n$ = 16000; $M_w$=26000
$T_g$ = 225°C $T_d$ = 428°C (Beginn der Gewichtsabnahme in Luft, Heizrate 10 K/min).

Die Polymeren aus den Beispielen 5-7 sind bei Raumtemperatur löslich in N-Methylpyrrolidon, Dimethylsulfoxid, THF und Chloroform. DSC-Messungen (DSC7, Perkin Elmer) zeigen die angegebenen Glasübergangstemperaturen, jedoch keinen Hinweis auf Teilkristallinität der Polymeren. Die angegebenen Molmassen wurden durch GPC (Polystyroleichung, THF als Eluent) ermittelt.

Beispiele 8-16

Analog zu Beispiel 1 wurden jeweils 0,44 mol der Fluor-Komponente BFBPI mit 0,44 mol Bishydroxykomponente umgesetzt. Die Struktur der erhaltenen Polymere, der Brechungsindex $n_D$, ihre inhärente Viskosität (gemessen in $CHCl_3$) und ihr Glasumwandlungspunkt $T_g$ sind in den Tabellen 1 und 2 angegeben.

Beispiel 17

Beispiel 1 wurde wiederholt. Jedoch wurde anstelle der Fluor-Komponente (BFBPI) 0,44 mol der entsprechenden

Chlorverbindung 4,6-Bis(4-chlorbenzoyl)1,3,3-trimethyl-1-phenylindan zusammen mit 0,146 mol KF eingesetzt.

Es entsteht das Polymer von Beispiel 1 mit einem $T_g$ von 200°C und einer inhärenten Viskosität von 0,36 dl/g (Chloroform; 0,1 g/dl; 25°C).

Tabelle 1

| | Inh.Visk. (dl/g) | Tg (°C) |
|---|---|---|
| Beispiel 8 (n = 1.616)<br>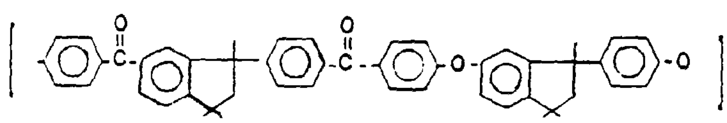 | 0,54 | 210,5 |
| Beispiel 9 (n = 1.614)<br>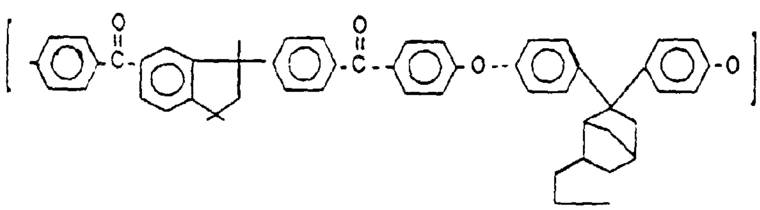 | 0,37 | 231,0 |
| Beispiel 10 (n = 1.625)<br>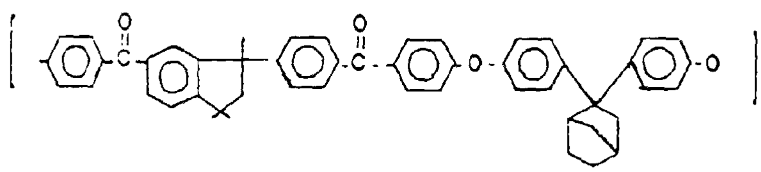 | 0,23 | 215,5 |
| Beispiel 11 (n = 1.644)<br>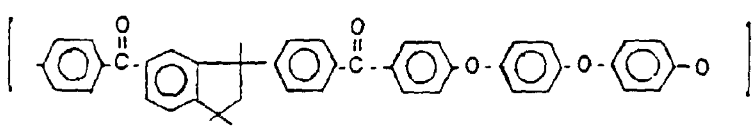 | 0,32 | 187,0 |
| Beispiel 12 (n = 1.627)<br>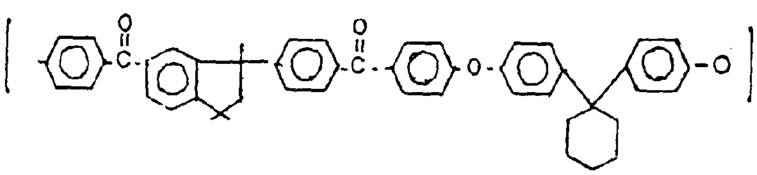 | 0,65 | 204,5 |

Tabelle 2

| | Inh.Visk. (dl/g) | Tg (°C) |
|---|---|---|
| Beispiel 13 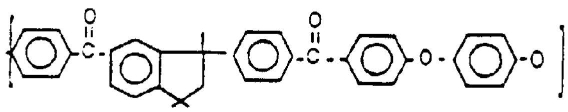 | 0,42 | 171,5 |
| Beispiel 14 (n = 1.639) 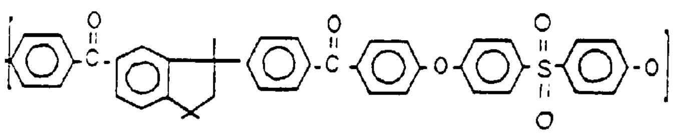 | 0,31 | 216,0 |
| Beispiel 15 (n = 1.646) 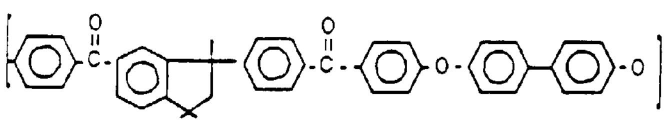 | 0,54 | 195,0 |
| Beispiel 16 (n = 1.659) 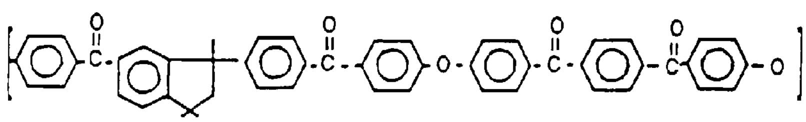 | 0,36 | 190,5 |

## Patentansprüche

1. Polymere Ether mit Phenylindan-Einheiten und polaren Gruppen, dadurch gekennzeichnet, daß sie die allgemeine Formel

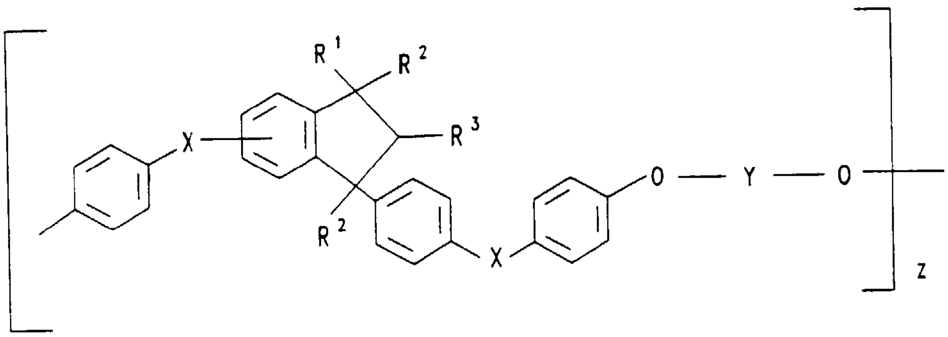

aufweisen, worin

X = CO oder $SO_2$,
Y = einen divalenten Heteroarylen-Rest oder Arylen-Rest oder eine zweiwertige Gruppe aus zwei über eine Brücke miteinander verbundenen Arylresten,
$R^1$= $CH_3$; $R^2 = R^3$ = H oder
$R^2$= $CH_3$; $R^1 = R^3$ = ($C_1$ bis $C_{16}$)-Alkyl oder Aryl oder
$R^1$= $CH_3$; $R^3$ = H; $R^2$ = ($C_1$ bis $C_{16}$)-Alkyl oder Aryl

bedeuten.

**2.** Polymerer Ether gemäß Anspruch 1, dadurch gekennzeichnet, daß der zweiwertige Arylenrest Y sich von einem Phenylindan ableitet.

**3.** Polymerer Ether gemäß Anspruch 2, dadurch gekennzeichnet, daß Y für

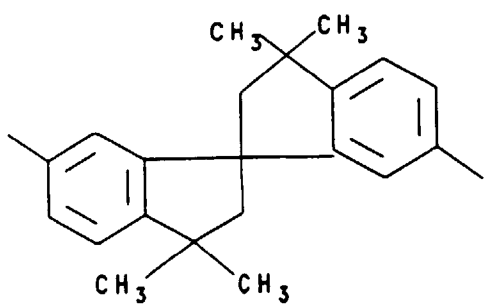

steht.

**4.** Polymerer Ether gemäß Anspruch 2, dadurch gekennzeichnet, daß Y für

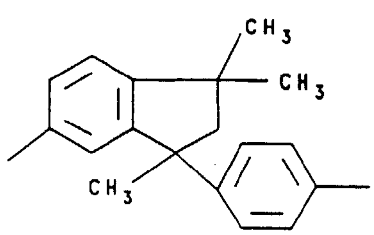

steht.

**5.** Polymerer Ether gemäß Anspruch 1, dadurch gekennzeichnet, daß Y ausgewählt ist aus den Resten:

$Y^1$ 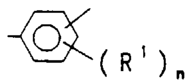

$Y^2$ 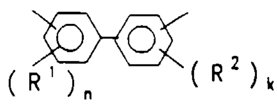

$Y^3$ 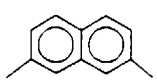 $Y^4$ 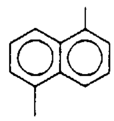 $Y^5$ 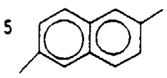

$Y^6$ 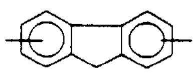 $Y^7$ 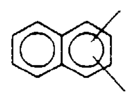

$Y^8$ 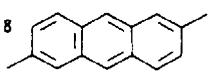 $Y^9$ 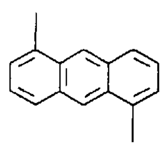 $Y^{10}$ 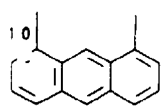 $Y^{11}$ 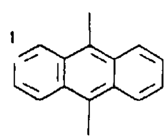

worin
$R^1$ und $R^2$ gleich oder verschieden sind und Halogen, $C_1$-$C_4$-Alkyl- oder $C_1$-$C_4$-Alkoxy-Gruppen oder eine Phenylgruppe darstellen, k und n gleich oder verschieden sind und Null oder eine ganze Zahl von 1 bis 4 bedeuten.

**6.** Polymerer Ether gemäß Anspruch 1, dadurch gekennzeichnet, daß Y gleich

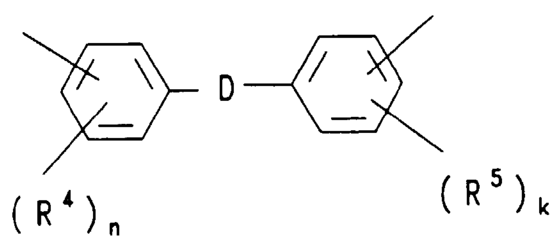

ist, worin $R^4$ und $R^5$ gleich oder verschieden sind und Halogen, $C_1$-$C_4$-Alkyl- oder $C_1$-$C_4$-Alkoxy-Gruppen darstellen, k und n gleich oder verschieden sind und Null oder eine ganze Zahl von 1 bis 4 bedeuten und D eine Ketogruppe oder einen Kohlenwasserstoff-Rest bedeutet, der durch Trifluor-methylgruppen substituiert sein kann.

**7.** Polymere Ether gemäß Anspruch 6, dadurch gekennzeichnet, daß D ausgewählt ist aus:

$D^1$ $-CH_2-$, $D^2$ $-C(CH_3)_2-$, $D^3$ $-C(CF_3)_2-$

$D^4$ 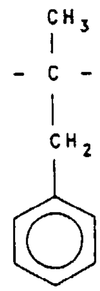 $D^5$ 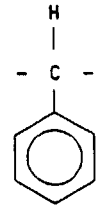 $D^6$ 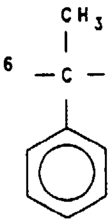

$D^7$ 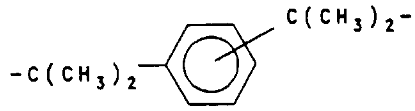 (m oder p)

$D^8$ 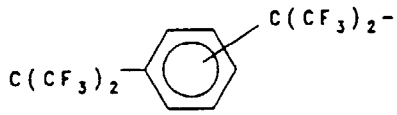 (m oder p)

$D^9$ 1,1-Cyclohexylen

$D^{10}$ 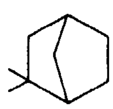 $D^{11}$ 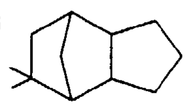 $D^{12}$ 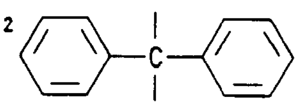

$D^{13}$ 9,9-Fluorenylen

**8.** Polymerer Ether gemäß Anspruch 1, dadurch gekennzeichnet, daß Y gleich

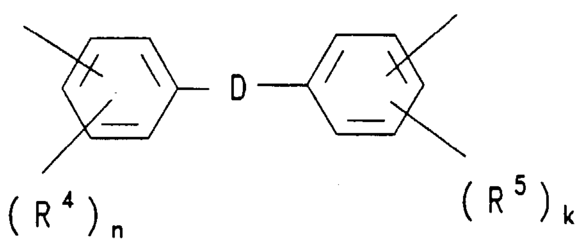

worin

$R^4$ und $R^5$ gleich oder verschieden sind und Halogen, $C_1$-$C_4$-Alkyl- oder $C_1$-$C_4$-Alkoxy-Gruppen darstellen, k und n gleich oder verschieden sind und Null oder eine ganze Zahl von 1 bis 4 bedeuten und D einen Heterocyclus darstellt.

**9.** Polymerer Ether gemäß Anspruch 8, dadurch gekennzeichnet, daß D ein Furylen-, Oxadiazolylen- oder Thiadiazolylen-Rest ist.

**10.** Polymerer Ether gemäß Anspruch 1, dadurch gekennzeichnet, daß Y gleich

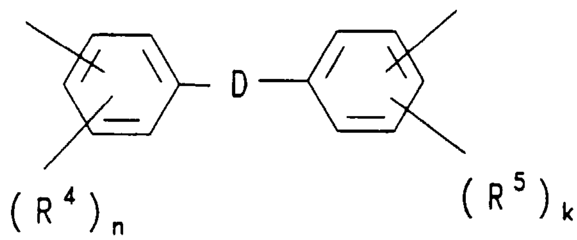

worin

$R^4$ und $R^5$ gleich oder verschieden sind und Halogen, $C_1$-$C_4$-Alkyl- oder $C_1$-$C_4$-Alkoxy-Gruppen darstellen, k und n gleich oder verschieden sind und Null oder eine ganze Zahl von 1 bis 4 bedeuten und D aus Schwefel und/oder Sauerstoff besteht.

**11.** Polymerer Ether gemäß Anspruch 10, dadurch gekennzeichnet, daß D ausgewählt ist aus:

$D^{15}$ -O-,

$D^{16}$ -S-,

$D^{17}$ -SO-,

$D^{18}$ $-SO_2-$.

**12.** Polymere Ether gemäß Anspruch 1, gekennzeichnet durch die allgemeine Formel

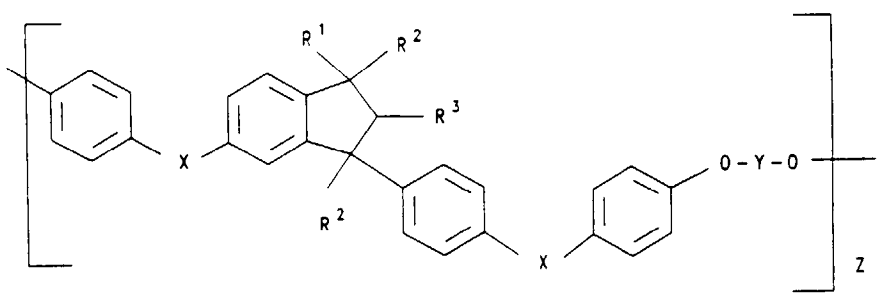

**13.** Polymere Ether gemäß Anspruch 1, dadurch gekennzeichnet, daß Y ausgewählt ist aus den zweiwertigen Heteroarylen-Resten 2,5-Pyridylen, 2,5-Furylen, 2,5-Oxadiazolylen, 2,5-Thiadiazolylen, 2,5-Oxazolylen.

**14.** Verfahren zur Herstellung eines polymeren Ethers gemäß Anspruch 1, dadurch gekennzeichnet, daß man eine Halogenverbindung der Formel (II)

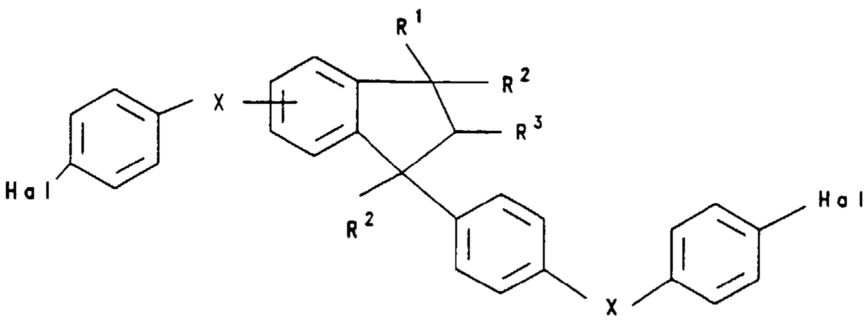

wobei Hal für Chlor, Fluor, Brom oder Jod steht und X, $R^1$, $R^2$ und $R^3$ die in Anspruch 1 angegebene Bedeutung haben, mit der äquimolaren Menge einer aromatischen oder heteroaromatischen Dihydroxyverbindung HO-Y-OH, wobei Y die in Anspruch 1 angegebene Bedeutung aufweist, in einem aprotisch-dipolaren Lösungsmittel umsetzt.

**15.** Verwendung der polymeren Ether gemäß Anspruch 1 zur Herstellung von Elementen für optische Systeme.

**16.** Zwischenprodukt der Formel

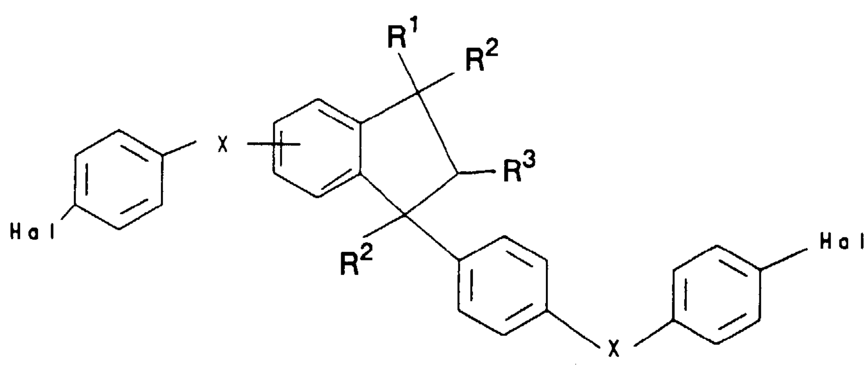

wobei Hal für F, Cl, Br oder J steht und X, $R^1$, $R^2$, $R^3$ die in Anspruch 1 angegebene Bedeutung aufweisen.

**17.** Zwischenprodukt gemäß Anspruch 16 der Formel

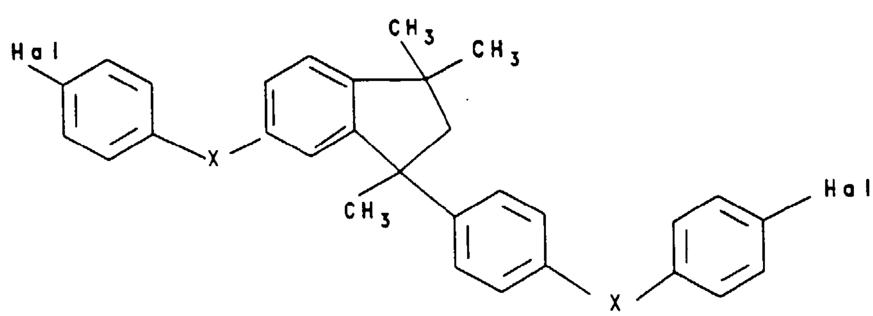

**18.** Verfahren zur Herstellung des Zwischenprodukts gemäß Anspruch 17, dadurch gekennzeichnet, daß man die Verbindung

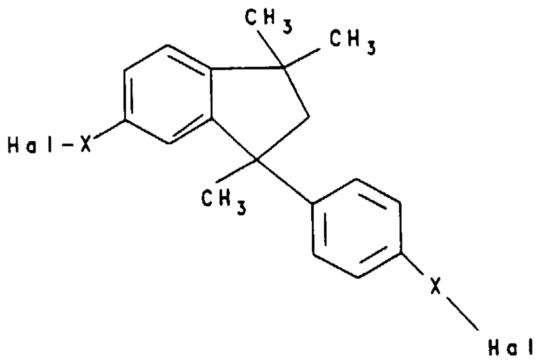

mit überschüssigem Halogenbenzol $C_6H_5$-Hal in Gegenwart einer Lewis-Säure, wie z.B. $AlCl_3$ umsetzt.

**19.** Verfahren zur Herstellung des Zwischenprodukts gemäß Anspruch 17, dadurch gekennzeichnet, daß man 1,1,3-Trimethyl-3-phenylindan mit einem Säurehalogenid der Formel Hal-p-$C_6H_4$-X-$Hal^1$, wobei Hal und $Hal^1$ unabhängig voneinander für Cl oder F und X für CO oder $SO_2$ stehen, in einem inerten Lösungsmittel in Gegenwart von $AlCl_3$ umsetzt und man das Rohprodukt umkristallisiert.

## Claims

**1.** A polymeric ether containing phenylindane units and polar groups, which has the formula

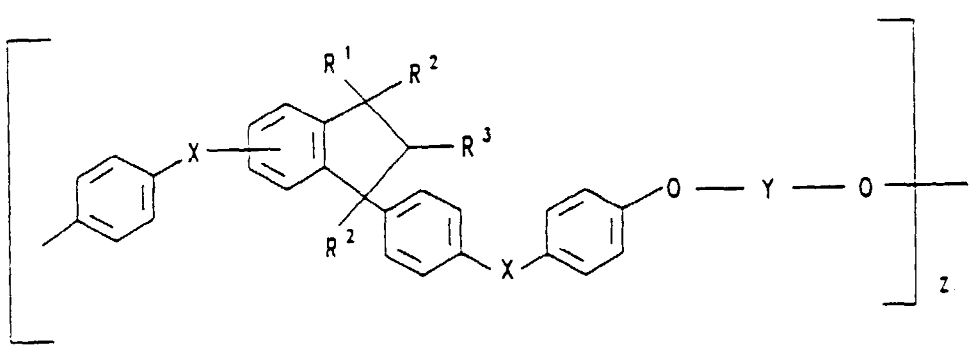

in which

X = CO or $SO_2$,
Y = a divalent heteroarylene radical or arylene radical or a divalent group consisting of two aryl radicals bonded to one another via a bridge,
$R^1$ = $CH_3$; $R^2 = R^3$ = H or
$R^2$ = $CH_3$; $R^1 = R^3$ = ($C_1$ to $C_{16}$)-alkyl or aryl, or
$R^1$ = $CH_3$; $R^3$ = H; $R^2$ = ($C_1$ to $C_{16}$)-alkyl or aryl.

**2.** A polymeric ether as claimed in claim 1, wherein the divalent arylene radical Y is derived from a phenylindane.

**3.** A polymeric ether as claimed in claim 2, wherein Y is

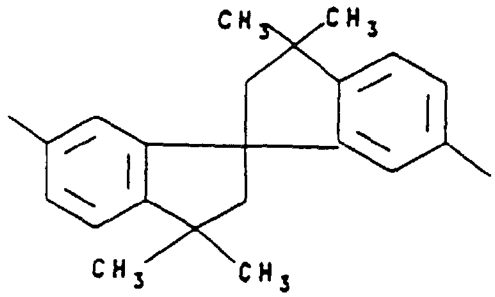 .

**4.** A polymeric ether as claimed in claim 2, wherein Y is

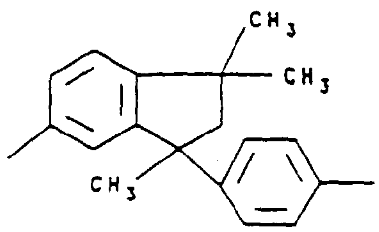 .

**5.** A polymeric ether as claimed in claim 1, wherein Y is selected from the radicals:

$Y^1$ 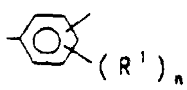

$Y^2$ 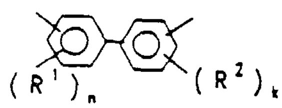

$Y^3$ 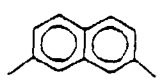 $Y^4$ 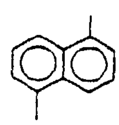 $Y^5$ 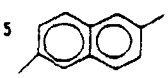

$Y^6$ 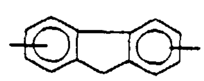 $Y^7$ 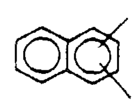

$Y^8$ 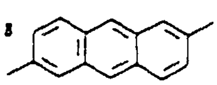 $Y^9$ 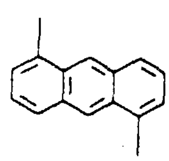 $Y^{10}$ 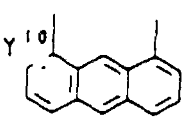 $Y^{11}$ 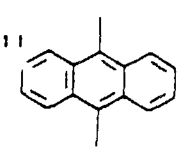

in which

$R^1$ and $R^2$ are identical or different and are halogen, $C_1$-$C_4$-alkyl or $C_1$-$C_4$-alkoxy groups or a phenyl group, and k and n are identical or different and are zero or an integer from 1 to 4.

6. A polymeric ether as claimed in claim 1, wherein Y is

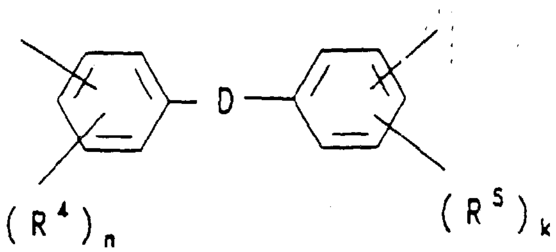

in which $R^4$ and $R^5$ are identical or different and are halogen, $C_1$-$C_4$-alkyl or $C_1$-$C_4$-alkoxy groups, k and n are identical or different and are zero or an integer from 1 to 4, and D is a keto group or a hydrocarbon radical, which may be substituted by trifluoromethyl groups.

7. A polymeric ether as claimed in claim 6, wherein D is selected from:

$D^1$ $-CH_2-$, $D^2$ $-C(CH_3)_2-$, $D^3$ $-C(CF_3)_2-$

$D^4$ 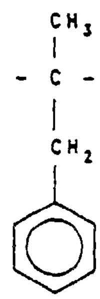 $D^5$ 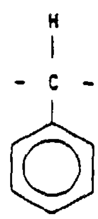 $D^6$ 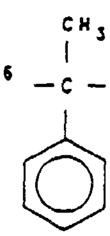

$D^7$ 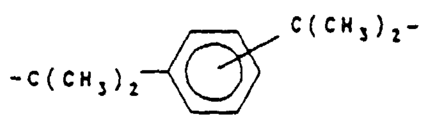 (m or p)

$D^8$ 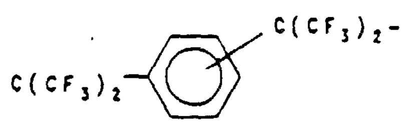 (m or p)

$D^9$ 1,1-cyclohexylene

$D^{10}$ 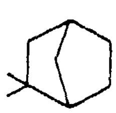 $D^{11}$ 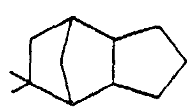 $D^{12}$ 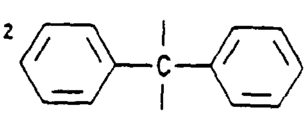

$D^{13}$ 9,9-fluorenylene.

8. A polymeric ether as claimed in claim 1, wherein Y is

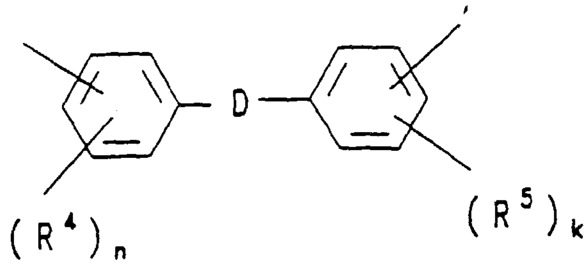

in which
$R^4$ and $R^5$ are identical or different and are halogen, $C_1$-$C_4$-alkyl or $C_1$-$C_4$-alkoxy groups, k and n are identical or different and are zero or an integer from 1 to 4, and D is a heterocyclic ring.

**9.** A polymeric ether as claimed in claim 8, wherein D is a furylene, oxadiazolylene or thiadiazolylene radical.

**10.** A polymeric ether as claimed in claim 1, wherein Y is

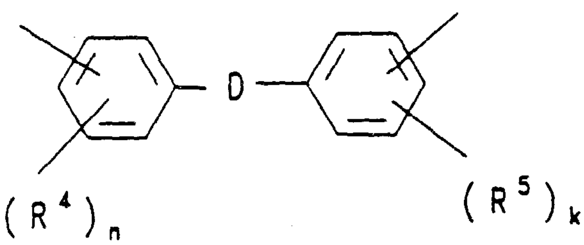

in which
$R^4$ and $R^5$ are identical or different and are halogen, $C_1$-$C_4$-alkyl or $C_1$-$C_4$-alkoxy groups, k and n are identical or different and are zero or an integer from 1 to 4, and D comprises sulfur and/or oxygen.

**11.** A polymeric ether as claimed in claim 10, wherein D is selected from:

| | |
|---|---|
| $D^{15}$ | -O-, |
| $D^{16}$ | -S-, |
| $D^{17}$ | -SO- and |
| $D^{18}$ | $-SO_2-$. |

**12.** A polymeric ether as claimed in claim 1 which has the formula

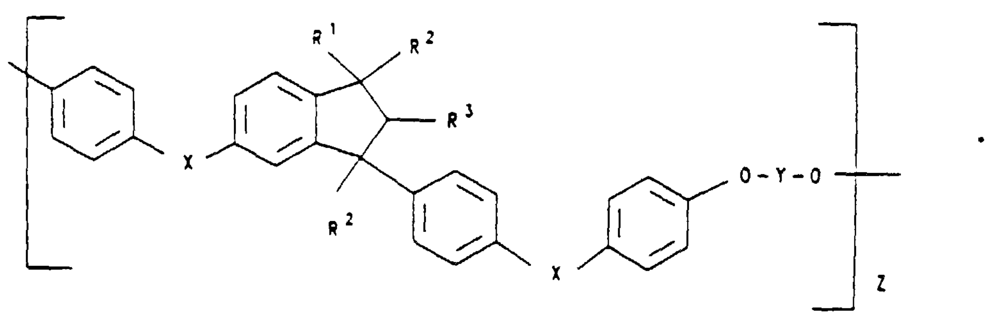

**13.** A polymeric ether as claimed in claim 1, wherein Y is selected from the divalent heteroarylene radicals 2,5-pyridylene, 2,5-furylene, 2,5-oxadiazolylene, 2,5-thiadiazolylene and 2,5-oxazolylene.

**14.** A process for the preparation of a polymeric ether as claimed in claim 1, which comprises reacting a halogen

compound of the formula (II)

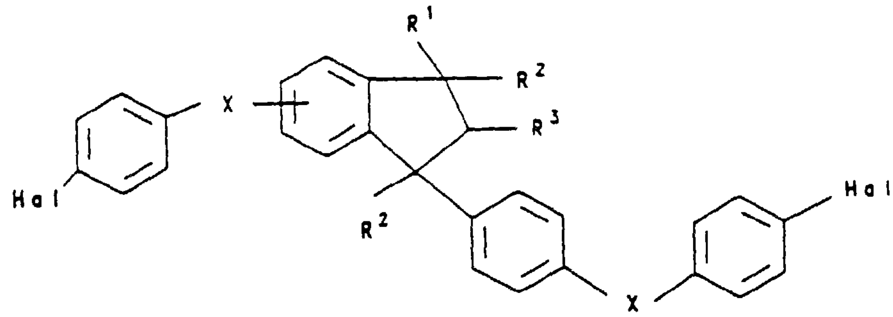

where Hal is chlorine, fluorine, bromine or iodine, and X, $R^1$, $R^2$ and $R^3$ are as defined in claim 1, with an equimolar amount of an aromatic or heteroaromatic dihydroxyl compound HO-Y-OH where Y is as defined in claim 1, in an aprotic, dipolar solvent.

**15.** A method of using a polymeric ether as claimed in claim 1 for the preparation of elements for optical systems.

**16.** An intermediate of the formula

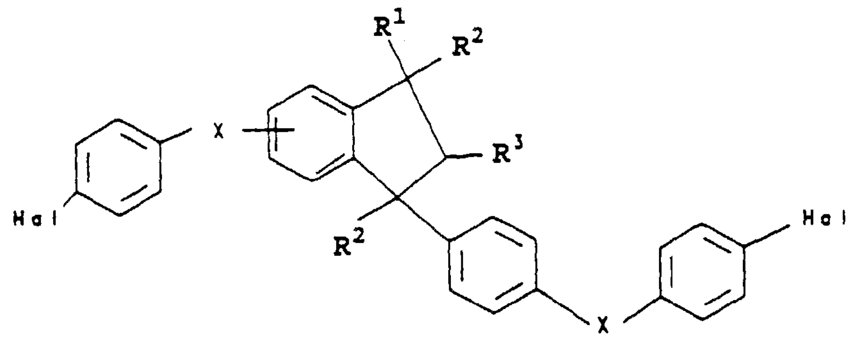

where Hal is F, Cl, Br or I, and X, $R^1$, $R^2$ and $R^3$ are as defined in claim 1.

**17.** An intermediate as claimed in claim 16, of the formula

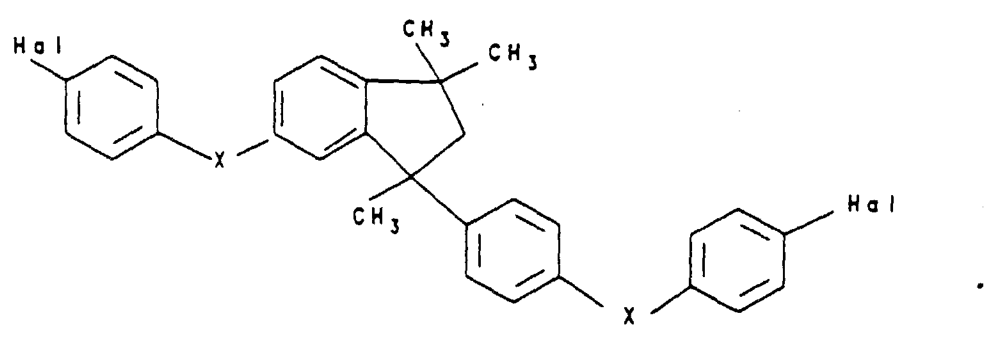

**18.** A process for the preparation of an intermediate as claimed in claim 17, which comprises reacting the compound

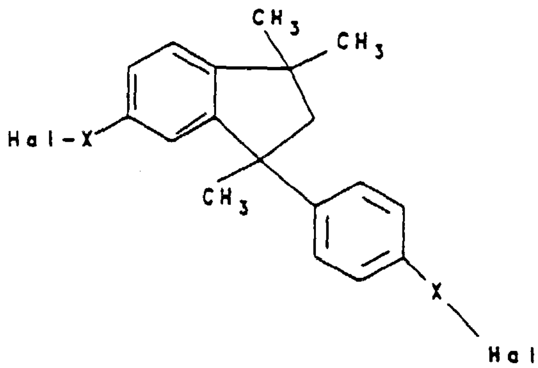

with excess halobenzene $C_6H_5$-Hal in the presence of a Lewis acid, such as, for example, $AlCl_3$.

**19.** A process for the preparation of an intermediate as claimed in claim 17, which comprises reacting 1,1,3-trimethyl-3-phenylindane with an acid halide of the formula Hal-p-$C_6H_4$-X-$Hal^1$, where Hal and $Hal^1$, independently of one another, are Cl or F, and X is CO or $SO_2$, in an inert solvent in the presence of $AlCl_3$, and recrystallizing the crude product.

**Revendications**

**1.** Ethers polymères comportant des motifs phénylindane et des groupes polaires, caractérisés en ce qu'ils ont la formule générale

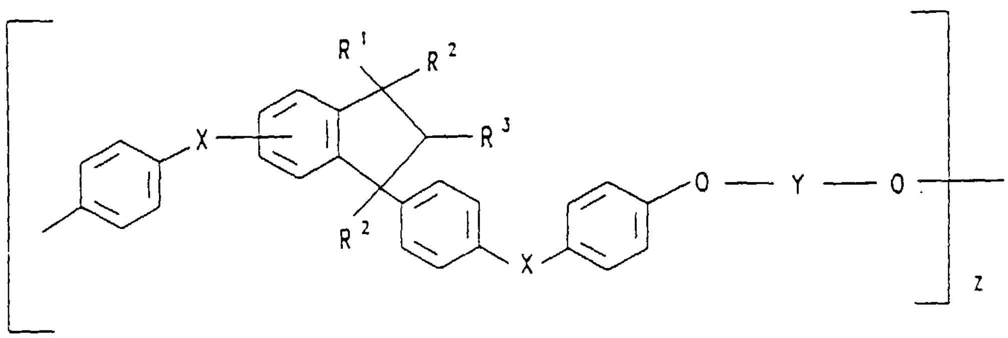

dans laquelle

X = CO ou $SO_2$,
Y est un radical hétéroarylène ou un radical arylène divalent ou un groupe divalent constitué de deux résidus aryle liés l'un à l'autre par un pont,
$R^1 = CH_3$; $R^2 = R^3 = H$ ou
$R^2 = CH_3$; $R^1 = R^3$ = (alkyle en $C_1$-$C_{16}$) ou aryle,
$R^1 = CH_3$ ; $R^3 = H$ ; $R^2$ = (alkyle en $C_1$-$C_{16}$) ou aryle.

**2.** Ether polymère selon la revendication 1, caractérisé en ce que le résidu arylène divalent Y dérive d'un phénylindane.

**3.** Ether polymère selon la revendication 2, caractérisé en ce que Y est

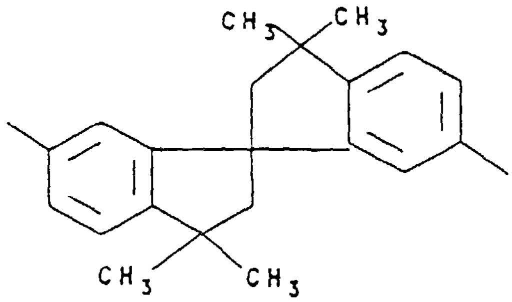

4. Ether polymère selon la revendication 2, caractérisé en ce que Y est

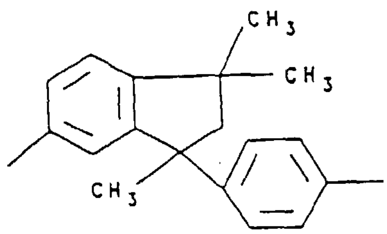

5. Ether polymère selon la revendication 1, caractérisé en ce que Y est choisi parmi les radicaux suivants :

$Y^1$ 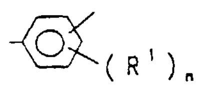

$Y^2$ 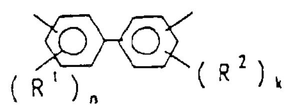

$Y^3$ 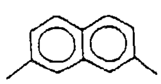 $Y^4$ 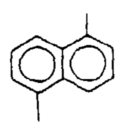 $Y^5$ 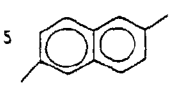

$Y^6$ 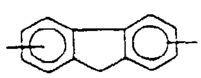 $Y^7$ 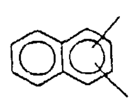

$Y^8$ 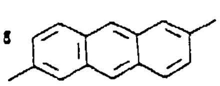 $Y^9$ 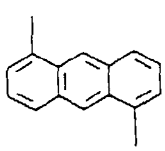 $Y^{10}$ 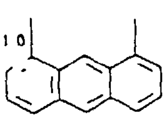 $Y^{11}$ 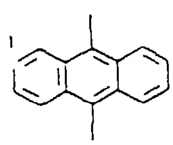

où

$R^1$ et $R^2$ sont identiques ou différents et représentent des halogènes, des groupes alkyle en $C_1$-$C_4$ ou alcoxy en $C_1$-$C_4$ ou un groupe phényle, k et n sont identiques ou différents, et n vaut zéro ou est un nombre entier de 1 à 4.

**6.** Ether polymère selon la revendication 1, caractérisé en ce que Y est

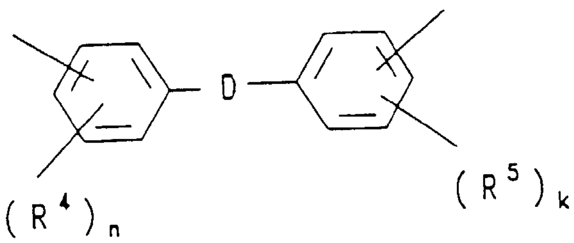

où $R^4$ et R5 sont identiques ou différents et représentent des halogènes , des groupes alkyle en $C_1$-$C_4$ ou alcoxy en $C_1$-$C_4$, k et n sont identiques ou différents et valent zéro ou représentent un nombre entier de 1 à 4, et D est un groupe céto ou un résidu hydrocarboné pouvant être substitué par des groupes trifluorométhyle.

**7.** Ether polymère selon la revendication 6, caractérisé en ce que D est choisi parmi les radicaux suivants

$D^1$ $-CH_2-$ , $D^2$ $-C(CH_3)_2-$ , $D^3$ $-C(CF_3)_2-$

$D^4$ 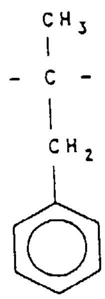 $D^5$ 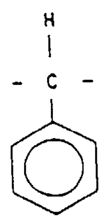 $D^6$ 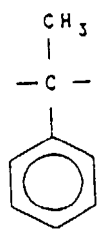

$D^7$ 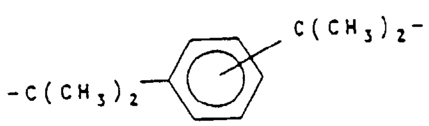 (m ou p)

$D^8$ 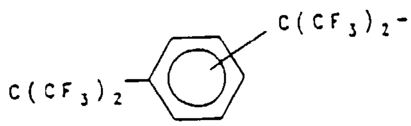 (m ou p)

$D^9$ 1,1-cyclohexylène

D10 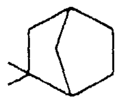 D11 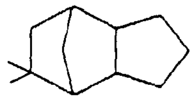 D12 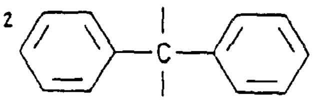

D13 9,9-fluorénylène

**8.** Ether polymère selon la revendication 1, caractérisé en ce que Y est

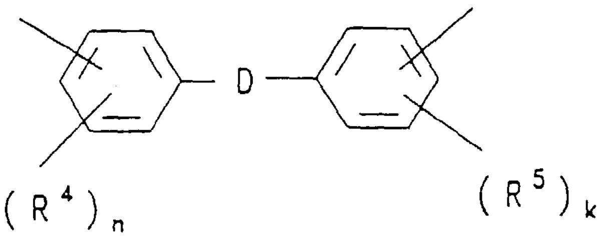

où $R^4$ et $R^5$ sont identiques ou différents et représentent des halogènes, des groupes alkyle en $C_1$-$C_4$ ou alcoxy en $C_1$-$C_4$, k et n sont identiques ou différents et valent zéro ou un nombre entier de 1 à 4, et D est un radical hétérocyclique.

**9.** Ether polymère selon la revendication 8, caractérisé en ce que D est un radical furylène, oxadiazolylène ou thiadiazolylène.

**10.** Ether polymère selon la revendication 1, caractérisé en ce que Y est

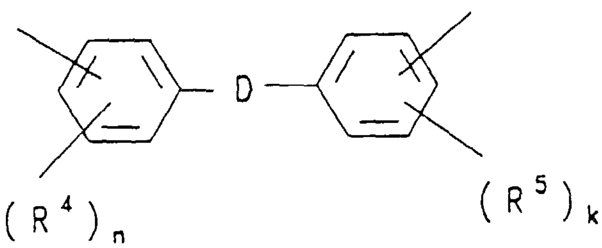

où $R^4$ et $R^5$ sont identiques ou différents et représentent des halogènes, des groupes alkyle en $C_1$-$C_4$ ou alcoxy en $C_1$-$C_4$, k et n sont identiques ou différents et valent zéro ou sont des nombres entiers de 1 à 4, et D est choisi parmi le soufre et/ou l'oxygène.

**11.** Ether polymère selon la revendication 10, caractérisé en ce que D est choisi parmi les radicaux suivants :

D15 -O-,
D16 -S-,
D17 -SO-,
D18 $-SO_2-$.

**12.** Ether polymère selon la revendication 1, caractérisé par la formule générale

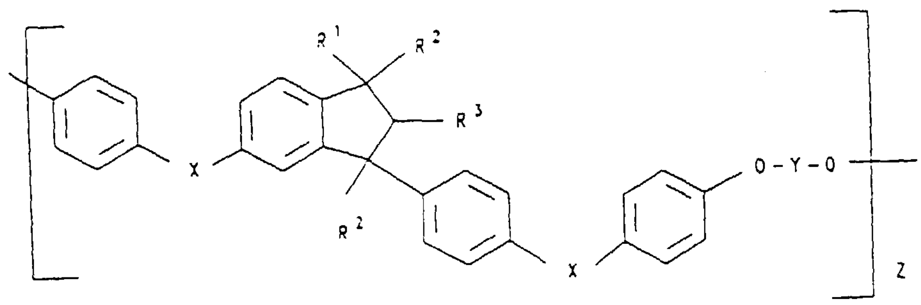

**13.** Ether polymère selon la revendication 1, caractérisé en ce que Y est choisi parmi les radicaux hétéroarylènes divalents 2, 5-pyridylène, 2,5-furylène, 2,5-oxadiazolylène, 2,5-thiadiazolylène, 2,5-oxazolylène.

**14.** Procédé pour préparer un éther polymère selon la revendication 1, caractérisé en ce qu'on fait réagir dans un solvant aprotique dipolaire un composé halogéné de formule (II)

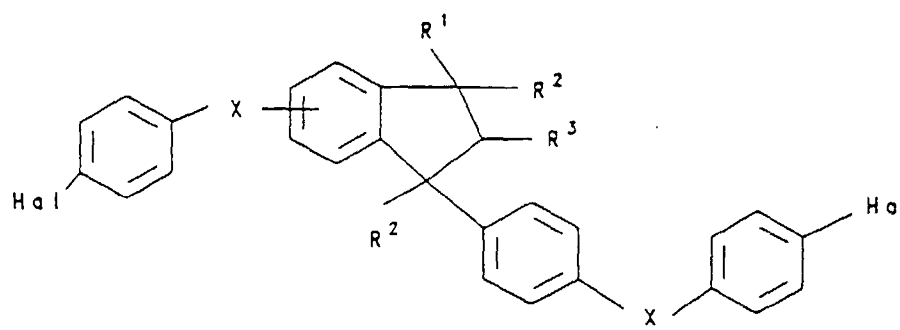

dans laquelle Hal est le chlore, le fluor, le brome ou l'iode, et X, $R^1$, $R^2$ et $R^3$ ont les significations données dans la revendication 1,

avec une quantité équimolaire d'un composé dihydroxylé aromatique ou hétéroaromatique HO-Y-OH, où Y a les significations données dans la revendication 1.

**15.** Utilisation des éthers polymères selon la revendication 1 pour préparer des éléments pour systèmes optiques.

**16.** Produit intermédiaire de formule

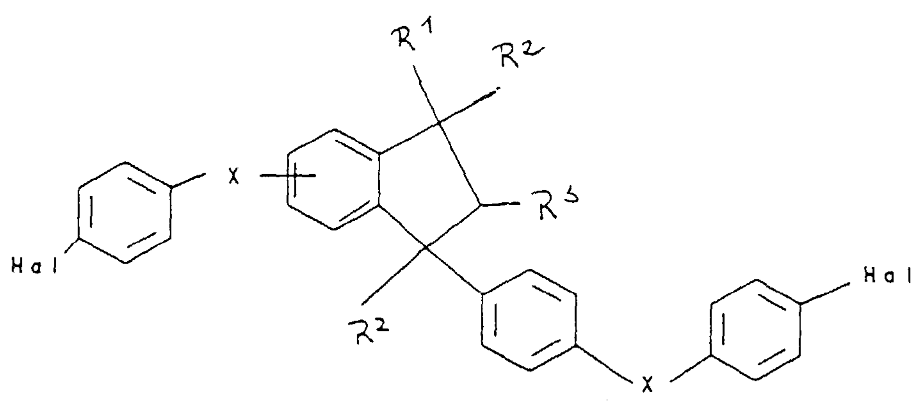

dans laquelle Hal représente F, Cl, Br ou I, et X, $R^1$, $R^2$ et $R^3$ ont les significations données dans la revendication 1.

**17.** Produit intermédiaire selon la revendication 16, ayant la formule suivante :

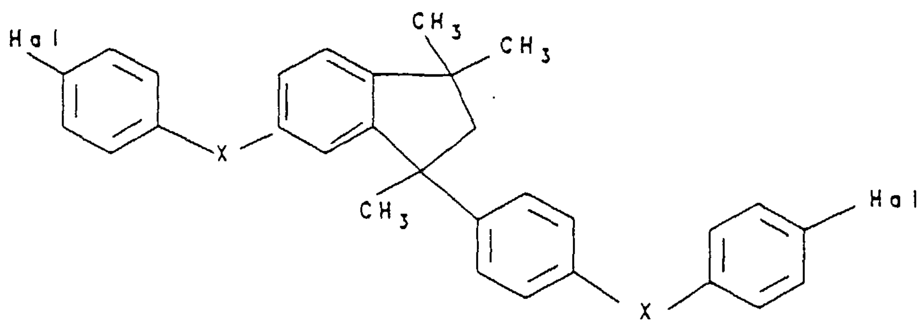

**18.** Procédé pour préparer le produit intermédiaire selon la revendication 17, caractérisé en ce qu'on fait réagir le composé

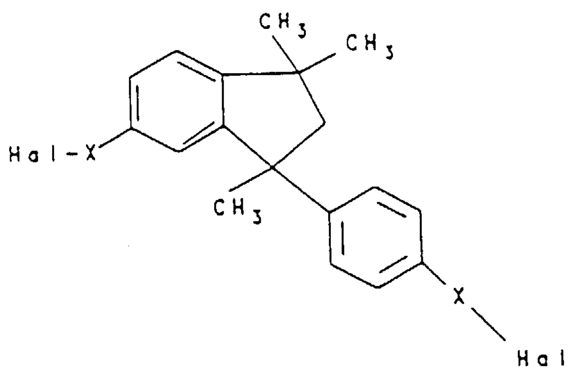

avec un halogénobenzène $C_6H_5$-Hal en excès, en présence d'un acide de Lewis, tel par exemple que $AlCl_3$.

**19.** Procédé pour préparer le produit intermédiaire selon la revendication 17, caractérisé en ce qu'on fait réagir dans un solvant inerte en présence d'$AlCl_3$ du 1,1,3-triméthyl-3-phénylindane avec un halogénure d'acyle de formule Hal-p-$C_6H_4$-X-$Hal^1$, où Hal et $Hal^1$ représentent, indépendamment l'un de l'autre, Cl ou F, et X est CO ou $SO_2$, dans un solvant inerte en présence d'$AlCl_3$, et que l'on recristallise le produit brut.